# EUROPÄISCHE PATENTSCHRIFT

(11) **EP 1 174 154 B1**
(45) Veröffentlichungstag und Bekanntmachung des Hinweises auf die Patenterteilung: **11.06.2003**
(21) Anmeldenummer: 01117450.5
(22) Anmeldetag: 19.07.2001
(51) Int. Cl.: A61L 2/26

(54) **"Blindcontainer" zur Reduzierung des Arbeitsvolumens eines Sterilisierapparates**
Dummy container for reduction of the working volume of steriliser
Accessoire réduisant le volume utile d'un stérilisateur

(30) Priorität: 19.07.2000 DE 10035082
(43) Veröffentlichungstag der Anmeldung: 23.01.2002
(73) Patentinhaber: Sirona Dental Systems GmbH, 64625 Bensheim (DE)
(72) Erfinder: Landgraf, Hermann, 64653 Lorsch (DE); Oehme, Bernd, 55128 Mainz (DE); Schreiber, Jürgen, 64625 Bensheim (DE); Sutter, Ralf, 69469 Weinheim (DE)
(74) Vertreter: Sommer, Peter

(56) Entgegenhaltungen:
- DE-A- 4 134 093
- DE-A- 19 519 430
- DE-A- 19 643 669
- DE-A- 19 717 663
- US-A- 5 433 929

## Beschreibung

Die Erfindung bezieht sich auf eine Vorrichtung zur Reduzierung des Arbeitsvolumens eines Apparates zur chemischen oder thermischen Desinfektion oder Sterilisation von medizinischen, insbesondere zahnmedizinischen Gegenständen, entsprechend dem Oberbegriff des Patentanspruches 1.

In der Medizintechnik werden sowohl Desinfektionsverfahren als auch Sterilisationsverfahren angewendet. Die Verfahren und deren zur Durchführung vorgesehener Apparate sind bekannt und in verschiedenen Literaturstellen beschrieben, sodass auf diese hier nicht näher einzugehen ist.

Die genannten Verfahren können sowohl mit als auch ohne Vakuum, d.h. Evakuierung der Behandlungsskammer ablaufen.

Bei der Desinfektion/Sterilisation von medizinischen, insbesondere zahnmedizinischen Gegenständen ergibt sich in der Praxis häufig, dass die Charge der zu desinfizierenden oder zu sterilisierenden Gegenstände nicht ausreichend ist um die Behandlungskammer voll zu beladen, oder es besteht der Wunsch, zwischendurch eine kleinere Charge zu desinfizieren oder sterilisieren. In beiden Fällen muß der Behandlungsprozess mit nur teilweise befüllter Behandlungskammer durchgeführt werden. Energie- und Zeitaufwand sind dabei nahezu gleich einer Desinfektion/Sterilisation mit voller Beladung.

Sterilisationsapparate wie sie in der zahnärztlichen Praxis eingesetzt werden, haben in der Regel ein Kammervolumen von etwa 15 bis 20 Liter. Demgegenüber weisen Desinfektionsapparate, die in der Regel ähnlich einer Geschirrspülmaschine aufgebaut sind, ein sehr viel größeres Kammervolumen auf.

Sollen solche Desinfektions- /Sterilisationsapparate zur Schnelldesinfektion bzw. Schnellsterilisation eingesetzt werden, d. h. mit einer Beladung von geringerem Volumen und Gewicht als baulich vorgesehen, so muß dennoch das gesamte, also auch das ungenutzte Kammervolumen mit den notwendigen Betriebsmitteln für eine Desinfektion/Sterilisation versorgt werden.

Im Falle einer Dampfsterilisation unter Vakuum bedeutet dies, dass auch das ungenutzte Volumen evakuiert, aufgeheizt und mit Dampf beaufschlagt werden muß. In diesem, wie auch in anderen Fällen, läßt sich bei einer reduzierten Beladung kein nennenswerter Zeitgewinn gegenüber einem Prozess mit voller Beladung der Sterilisationskammer erzielen.

Um Sterilisationsgut wirtschaftlich auch in kleinen Mengen und in kürzerer Zeit sterilisieren zu können, wurde schon vorgeschlagen (EP 0 532 135 A1), außerhalb des Sterilisationsapparates, also extern der Sterilisationskammer, einen separaten Druckbehälter vorzusehen, in den die geringere Menge Sterilisationsgut eingebracht werden kann. Der separate Druckbehälter ist über eine Verbindungsleitung mit dem Sterilisationsapparat verbunden, wodurch er die zur Sterilisation erforderlichen Betriebsmittel erhalten kann.

Nachteilig bei dem bekannten Sterilisationsapparat ist unter anderem der erhöhte bauliche Aufwand, der zusätzlich beanspruchte Stellplatz und die durch die Verbindungsleitung entstehenden Verluste bei der Zuleitung des Dampfes und der Evakuierung des Druckbehälterraumes.

Der im Patentanspruch 1 angegeben Erfindung liegt die Aufgabe zugrunde, demgegenüber eine Verbesserung zu erzielen und eine Möglichkeit zu schaffen, auch kleinere Mengen an Behandlungsgut wirtschaftlich desinfizieren bzw. sterilisieren zu können ohne dass hierzu bauliche Veränderungen am Desinfektions- /Sterilisationsapparat vorgenommen werden müssen oder dass, wie bei dem vorgenannten Stand der Technik, ein separater Druckbehälter vorgesehen werden muß.

Gemäß der Erfindung kann der nicht ausgelastete Raum einer Desinfektions-/Sterilisationskammer mit ein oder mehreren Körpern aus desinfektions- bzw. sterilisationsbeständigem Material bestückt werden. Durch Anordnung solcher 'Blindcontainer' kann das Kammervolumen optimal auf die Menge des zu behandelnden Gutes abgestimmt werden. Ein handelsüblicher, auf ein bestimmtes Volumen ausgerichteter Desinfektions-/Sterilisationsapparat kann demnach sowohl mit voller Beladung als auch als 'Schnelldesinfektionsapparat' bzw. 'Schnellsterilisator' mit geringerer Beladung sehr wirtschaftlich betrieben werden.

Der 'Blindcontainer' ist dabei so beschaffen, dass er eine geringere Wärmekapazität als der ausgefüllte Raum aufweist. So können die Prozessphasen Vakuumziehen, Aufheizen und Trocknen bei einer Dampfsterilisation sehr viel schneller ablaufen als bisher. Nachdem weniger Raumvolumen aufgeheizt werden muß, wird sehr viel weniger Strom und auch weniger demineralisiertes Wasser zur Dampferzeugung verbraucht.

Den Erfordernissen der geringen Wärmekapazität kann entweder durch ein Vakuum im 'Blindcontainer', durch den Einsatz eines geeigneten Werkstoffes der über eine geringe Wärmekapazität verfügt, oder durch den Einsatz eines geeigneten, Wärme isolierenden Materials (schlechter Wärmeleiter) welches den 'Blindcontainer' umgibt, entsprochen werden.

Zwar ist die Verringerung eines zu behandelnden Raumvolumens grundsätzlich bekannt. Die DE 197 17 663 A1 betrifft ein Verfahren zur Reduzierung des Rauminhaltes in Verbindung mit der Einbringung von gasförmigen Medien in geschlossenen Räumen und Kammern zur Minimierung der einzusetzenden Wirkstoffe, sowie zur Minimierung des Energieund Zeitaufwandes. Das Verfahren ist vornehmlich zur Desinfektion bzw. Schädlingsbekämpfung von Räumen oder Kammern jeglicher Art vorgesehen. Das Verfahren basiert darauf, den zu behandelnden Rauminhalt durch Einbringen einer oder mehrerer flexibler Hüllen zu verkleinern, um so die Menge der einzubringenden Medien bzw. Wärmeträger zu verringern. Zielsetzung ist dabei, möglichst wenig Schadstoffe einzusetzen. Zur besseren Zirkulation sind um die Hülle(n) herum Abstandhalter (Noppen) angeordnet. In der DE 41 34 093 wird ein Verfahren zur Begasung eines Gebäudeinnenraumes, z. B. zum Zwecke einer Schädlingsbekämpfung von nicht mobilen Gegenständen in einer Kirche oder in einem Schloss oder zur Vorratsschutzbehandlung von Lebens-mitteln die in großvolumigen Lagerhallen eingelagert werden, beschrieben. Vor dem Einleiten des Gases wird in den Innenraum in dem sich die zu behandelnden Gegenstände befinden, ein Füllkörper eingebracht, der das Behandlungsgas aufnehmende Volumen des Gebäudeinnenraumes auf ein Restvolumen verkleinert. Als Füllkörper kommen aufblasbare Hohlkörper zur Anwendung. Es ist aber festzuhalten, dass auch bei näherer Betrachtung des keine Übereinstimmungen zwischen dem Erfindungsgegenstand und dem Gegenstand dieser Entgegenhaltungen vorhanden sind, insbesondere auch weil deren Zielsetzungen bzw. Aufgabestellungen unterschiedlich sind.

Vorteilhafterweise ist der 'Blindcontainer' als zumindest teilevakuierter Hohlkörper ausgebildet. Um ein Zusammenfallen des Behälters durch das Vakuum im Behälter oder durch den von außen einwirkenden Druck während des Behandlungsvorganges zu vermeiden, ist der Innenraum des Hohlkörpers mit Stützelementen, z. B. in Form eines Stützgerippes, versehen. Anstelle der Stützelemente kann der Hohlkörper auch mit einem geeigneten Schaumstoff gefüllt sein.

Der 'Blindcontainer' kann vorteilhafterweise außen mit einem Material geringer Wärmeleitfähigkeit, z. B. mit Keramikmaterial beschichtet sein.

Alternativ zu der vorgenannten Hohlkörperversion kann der 'Blindcontainer' auch aus einem desinfektionsbeständigen bzw. sterilisationsfesten Kunststoffblock bestehen. Der Kunststoffblock kann ein Schaumstoff, beispielsweise ein Siliconschaum, sein, der gegebenenfalls mit einer desinfektionsbeständigen bzw. sterilisationsfesten Folie überzogen ist.

Gemäß einer weiteren vorteilhaften Ausführung kann der 'Blindcontainer' so gestaltet sein, dass seine Größe, d.h. sein Volumen variierbar und damit dem nicht ausgelasteten freien Raum der Behandlungskammer bedarfsgerecht anpassbar ist. Das Gehäuse des 'Blindcontainers' kann beispielsweise mit einem Faltenbalg versehen sein, dessen Größe so bemessen ist, dass das Volumen des 'Blindcontainers' dem nicht ausgelasteten freien Raum der Behandlungskammer angepasst werden kann.

### Kurzbeschreibung der Zeichnung

Anhand der Zeichnung wird ein Ausführungsbeispiel der Erfindung näher erläutert. Es zeigen:
- Fig. 1: eine Frontansicht eines Sterilisationsapparates mit drei eingelegten Containern,
- Fig. 2: eine Seitenansicht des Sterilisationsapparates nach Figur 1 im Schnitt,

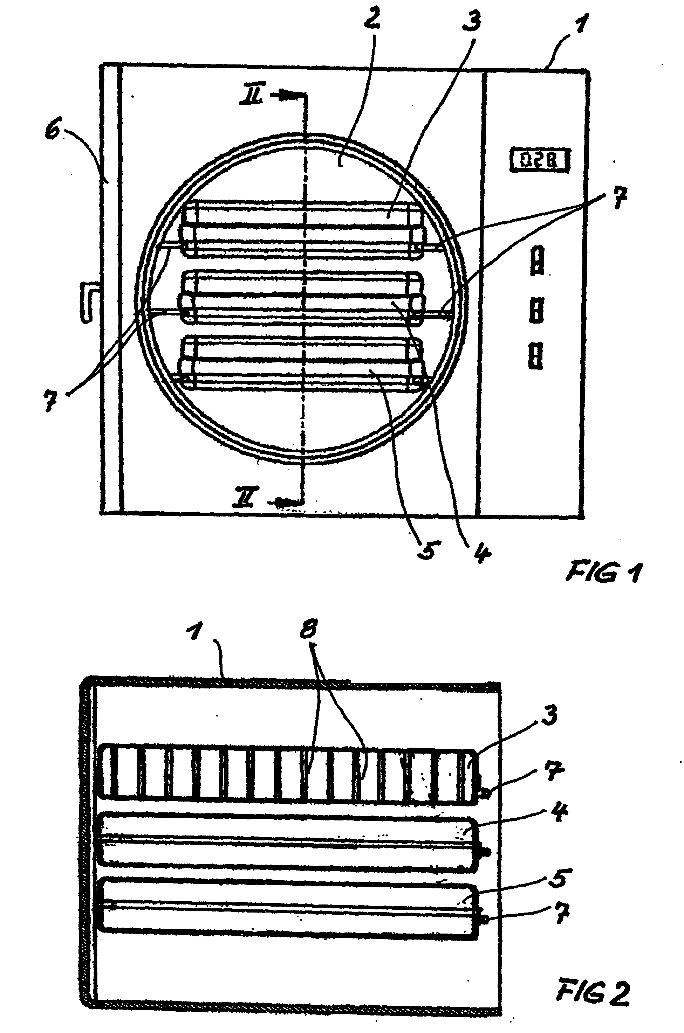

Die Figur 1 zeigt in einer Frontansicht einen Dampfsterilisationsapparat mit einem Gehäuse 1 in dem eine Sterilisationskammer 2 zur Aufnahme mehrerer Container 3, 4, 5 angeordnet ist. Die Sterilisationskammer 2 kann durch eine Tür 6 - hier im geöffneten Zustand gezeigt - dicht verschlossen werden. Im Gehäuse 1 sind ferner in üblicher Weise die zur Bereitstellung der Betriebsmittel sowie zur Steuerung des Sterilisationsprozesses erforderlichen Einrichtungen untergebracht.

In der hier zylindrisch ausgebildeten Sterilisationskammer 2 befinden sich seitlich Haltebügel 7 für die Container 3 bis 5 die ein einfaches und rasches Bestücken und Entnehmen der Container erlauben.

Von den drei Containern ist der obere Container 3 ein 'Blindcontainer' der nicht zur Aufnahme von Sterilisationsgut geeignet ist, während die beiden darunter befindlichen Container 4, 5 zur Beladung mit Sterilisiergut nach Art der bekannten Dental-Trays (Kassetten) nach DIN 13999 ausgeführt sind. Solche Normtrays sind üblicherweise zweigeteilt und bestehen aus einer Schale (Tablett) in die das zu sterilsierende Gut eingelegt wird, und einem Deckel. Beide, Schale und Deckel können perforiert oder unperforiert ausgebildet sein. Die Normtrays sind üblicherweise aus rostfreiem Stahl in den Abmessungen 188x289x40 mm ausgeführt.

Der 'Blindcontainer' 3 ist zweckmäßigerweise in denselben Maßen ausgeführt wie die oben genannten Normtrays, dient aber nicht dazu, Sterilisationsgut aufzunehmen, sondern das nicht genutzte Volumen der Sterilisationskammer 2 bei Bedarf, also bei nicht voller Beladung der Sterilisationskammer, aufzufüllen.

Der 'Blindcontainer' 3 kann unterschiedlich ausgebildet und auch aus verschiedenen Materialien gefertigt sein.

In einer ersten vorteilhaften Ausführung ist der 'Blindcontainer' als geschlossener, unperforierter Hohlkörper nach der oben erwähnten DIN 13999 ausgeführt. Der Hohlkörper kann aus sterilisationsfestem Kunststoff, z. B aus PEEK-Material, oder auch aus rostfreiem Metall, z. B aus Edelstahl, bestehen. Um den von außen wirkenden Druck während einer Sterilisation abzufangen, kann der Hohlkörper zur Abstützung der Wandungen mit diversen Stützelementen 8, z. B. in Form eines Stützgerippes versehen sein. Letzteres ist insbesondere dann notwendig, wenn der 'Blindcontainer' bei thermischen Sterilisationsprozessen eingesetzt werden soll und der Innenraum des 'Blindcontainers' unter Vakuum oder Teilvakuum steht. Durch das Evakuieren oder Teilevakuieren, welches zweckmäßigerweise bereits bei der Herstellung des 'Blindcontainers' erfolgen kann, erhält der 'Blindcontainer' eine sehr geringe Wärmeleitfähigkeit.

Denkbar ist es auch, den Hohlraum mit einem Material zu füllen, welches sterilisationsbeständig ist und eine sehr geringe Wärmekapazität aufweist. Hierfür kann beispielsweise ein Siliconschaum eingesetzt werden der Temperaturen über 135ºC verträgt.

Der 'Blindcontainer' kann alternativ auch aus einem Kunststoffblock bestehen der bereits in den erwähnten Konturen und Abmessungen gefertigt ist. Der 'Blindcontainer' bzw. der Kunststoffblock kann gegebenenfalls mit einer Schicht aus einem Material geringer Wärmeleitfähigkeit versehen sein, z. B. mit Keramik beschichtet oder mit einer geeigneten Folie überzogen sein.

Durch Einlegen ein oder mehrerer, auch verschieden gestalteter 'Blindcontainer' kann das nicht genutzte Volumen der Sterilisationskammer aufgefüllt werden ohne dass dieses aufgefüllte Volumen bzw. die entsprechende Masse im Verlauf des Sterilisationsprozesses selbst aufgeheizt zu werden braucht. Die Sterilisationskammer kann so durch die 'Blindcontainer' optimal an das Ladevolumen der Sterilisationskammer angepasst werden.

Die am vorbeschriebenen Ausführungsbeispiel aufgezeigten Merkmale lassen sich auch auf Ausführungen der eingangs erwähnten Desinfektionsapparate entsprechend anwenden.

### Bezugszeichenliste

- 1=: Gehäuse
- 2=: Sterilisationskammer
- 3=: Blindcontainer
- 4=: Container
- 5=: Container
- 6=: Tür
- 7=: Haltebügel
- 8=: Stützelemente

## Patentansprüche

1. Vorrichtung zur Reduzierung des Arbeitsvolumens eines Apparates zur chemischen oder thermischen Desinfektion oder Sterilisation von medizinischen, insbesondere zahnmedizinischen Gegenständen, die in einer Behandlungskammer (2), vorzugsweise in Containern (4, 5) eingelegt, behandelt werden, **gekennzeichnet durch** einen als 'Blindcontainer' ausgebildeten Körper (3) aus desinfektionsbeständigem bzw. sterilisationsfestem Material, der bei Bedarf in die Behandlungskammer (2) einlegbar ist und den zur Befüllung mit Behandlungsgut nicht ausgelasteten freien Raum ausfüllt, wobei der 'Blindcontainer' (3) so beschaffen ist, dass er während der chemischen oder thermischen Desinfektion oder Sterilisation eine geringere Wärmekapazität als der nicht ausgelastete freie Raum aufweist.

2. Vorrichtung nach Anspruch 1, **dadurch gekennzeichnet, daß** der 'Blindcontainer'(3) als Hohlkörper ausgebildet ist.

3. Vorrichtung nach Anspruch 2, **dadurch gekennzeichnet, daß** der Hohlkörper als mit mindestens einem Teilvakuum versehener Behälter ausgeführt ist.

4. Vorrichtung nach Anspruch 2, **dadurch gekennzeichnet, daß** der Hohlkörper mit Schaumstoff gefüllt ist.

5. Vorrichtung nach Anspruch 1, **dadurch gekennzeichnet, daß** der 'Blindcontainer' (3) aus einem desinfektionsbeständigen bzw. sterilisationsfesten Kunststoffblock besteht.

6. Vorrichtung nach Anspruch 5, **dadurch gekennzeichnet, daß** der 'Blindcontainer' (3) aus einem mit einer Folie überzogenen Schaumstoff besteht.

7. Vorrichtung nach Anspruch 3, **dadurch gekennzeichnet, daß** der Hohlkörper mit im Innenraum angeordneten Stützelementen (8) versehen ist.

8. Vorrichtung nach einem der Ansprüche 1 bis 7, **dadurch gekennzeichnet, daß** der 'Blindcontainer' (3) in seiner Größe variierbar gestaltet ist.

9. Vorrichtung nach einem der Ansprüche 1 bis 7, **dadurch gekennzeichnet, daß** der 'Blindcontainer' in Form und/oder Abmessungen denen von genormten dentalen Trays entspricht.

10. Vorrichtung nach einem der Ansprüche 1 bis 9, **dadurch gekennzeichnet, daß** der 'Blindcontainer' (3) außen mit einer Schicht aus einem Material mit geringer Wärmeleitfähigkeit versehen ist.

11. Vorrichtung nach einem der Patentansprüche 1 bis 10, in Verbindung mit einem Sterilisationsapparat, insbesondere einem Dampfsterilisationsapparat.

## Claims

1. Device for reducing the working volume of an apparatus for chemical or thermal disinfection or sterilization of medical articles, in particular dental articles, which, preferably placed in containers (4, 5), are treated in a treatment chamber (2), **characterized by** a body (3) which is designed as a "dummy container", is made of material that withstands disinfection or sterilization and, when required, can be placed in the treatment chamber (2) and fills the free space not occupied by articles to be treated, the nature of the "dummy container" (3) being such that, during the chemical or thermal disinfection or sterilization, it has a lower heat capacity than the unoccupied free space.

2. Device according to Claim 1, **characterized in that** the "dummy container" (3) is designed as a hollow body.

3. Device according to Claim 2, **characterized in that** the hollow body is designed as a vessel provided with at least a partial vacuum.

4. Device according to Claim 2, **characterized in that** the hollow body is filled with foam.

5. Device according to Claim 1, **characterized in that** the "dummy container" (3) consists of a block of plastic which withstands disinfection or sterilization.

6. Device according to Claim 5, **characterized in that** the "dummy container" (3) consists of a foam covered with a foil.

7. Device according to Claim 3, **characterized in that** the hollow body is provided with support elements (8) arranged in the inside.

8. Device according to one of Claims 1 to 7, **characterized in that** the "dummy container" (3) is designed so that its size can be varied.

9. Device according to one of Claims 1 to 7, **characterized in that** the shape and/or dimensions of the "dummy container" (3) correspond to those of standard dental trays.

10. Device according to one of Claims 1 to 9, **characterized in that** the "dummy container" (3) is provided on its outside with a layer of a material with low heat conductivity.

11. Device according to one of Patent Claims 1 to 10, in combination with a sterilization apparatus, in particular a steam sterilization apparatus.

## Revendications

1. Dispositif pour réduire le volume de travail d'un appareil de désinfection ou de stérilisation chimique ou thermique d'objets médicaux, plus particulièrement de médecine dentaire, qui sont traités dans une chambre de traitement (2), de préférence logés dans des récipients (4, 5), **caractérisé par** un corps (3) réalisé sous la forme d'un « récipient aveugle » constitué d'un matériau résistant à la désinfection ou résistant à la stérilisation qui peut, en cas de besoin, être introduit dans la chambre de traitement (2) et qui remplit l'espace libre non chargé destiné à être rempli de matériel à traiter, le « récipient aveugle » (3) étant réalisé de telle manière qu'il présente une capacité thermique inférieure à celle de l'espace libre non chargé pendant la désinfection ou la stérilisation chimique ou thermique.

2. Dispositif selon la revendication 1, **caractérisé en ce que** le « récipient aveugle » (3) est réalisé sous la forme d'un corps creux.

3. Dispositif selon la revendication 2, **caractérisé en ce que** le corps creux est réalisé sous la forme d'un réservoir muni au moins d'un vide partiel.

4. Dispositif selon la revendication 2, **caractérisé en ce que** le corps creux est rempli de mousse.

5. Dispositif selon la revendication 1, **caractérisé en ce que** le « récipient aveugle » (3) se compose d'un bloc en matière plastique résistant à la désinfection ou résistant à la stérilisation.

6. Dispositif selon la revendication 5, **caractérisé en ce que** le « récipient aveugle » (3) se compose d'une mousse recouverte d'un film.

7. Dispositif selon la revendication 3, **caractérisé en ce que** le corps creux est muni d'éléments supports (8) disposés dans son espace intérieur.

8. Dispositif selon l'une des revendications 1 à 7, **caractérisé en ce que** le « récipient aveugle » (3) est configuré de manière à pouvoir changer de taille.

9. Dispositif selon l'une des revendications 1 à 7, **caractérisé en ce que** la forme et/ou les dimensions du « récipient aveugle »(3) correspondent à celles des plateaux dentaires normalisés.

10. Dispositif selon l'une des revendications 1 à 9, **caractérisé en ce que** le « récipient aveugle » (3) est muni à l'extérieur d'une couche de matériau à faible conductivité thermique.

11. Dispositif selon l'une des revendications 1 à 10, combiné avec un appareil de stérilisation, plus particulièrement un appareil de stérilisation par vapeur.
